(19) **Europäisches Patentamt**

**European Patent Office**

**Office européen des brevets**

(11) Publication number : **0 529 898 A1**

(12) # EUROPEAN PATENT APPLICATION

(21) Application number : **92307474.4**

(22) Date of filing : **14.08.92**

(51) Int. Cl.⁵ : **A61K 31/485,** A61K 31/165, // (A61K31/485, 31:165)

(30) Priority : **16.08.91 US 747154**

(43) Date of publication of application : **03.03.93 Bulletin 93/09**

(84) Designated Contracting States : **DE ES GB IT**

(71) Applicant : **McNEIL-PPC, INC. Van Liew Avenue Milltown New Jersey 08850 (US)**

(72) Inventor : **Helzner, Eileen Cohler 505 Anthony Drive Plymouth Meeting, PA 19462 (US)**

(74) Representative : **Mercer, Christopher Paul Carpmaels & Ransford 43, Bloomsbury Square London WC1A 2RA (GB)**

(54) Potentiation of the antitussive effect of dextromethorphan with acetaminophen (paracetamol).

(57) A method of enhancing the antitussive effectiveness of dextromethorphan by administering to a patient an antitussive-effective amount of dextromethorphan with an antitussive potentiating amount of acetaminophen and improved antitussive pharmaceutical compositions consisting of the combination of dextromethorphan and acetaminophen.

EP 0 529 898 A1

## Field of the Invention

This invention relates to methods and compositions for enhancing the antitussive effectiveness of dextromethorphan. More particularly, this invention relates to the potentiation of the antitussive effectiveness of dextromethorphan by administering a potentiating effective amount of acetaminophen with dextromethorphan.

## Background of the Invention

Acetaminophen is a well-known clinically proven analgesic and antipyretic. Acetaminophen produces analgesia by elevation of the pain threshold and antipyresis through action on the hypothalamic heat-regulating center.

Acetaminophen provides effective analgesia in a wide variety of arthritic and rheumatic conditions involving musculoskeletal pain, as well as in other painful disorders such as headache, dysmenorrhea, myalgias and neuralgias. In addition, acetaminophen is indicated as an analgesic and antipyretic in diseases accompanied by discomfort and fever, such as the common cold and other viral infections. Acetaminophen is particularly well-suited as an analgesic-antipyretic in the presence of aspirin allergy, hemostatic disturbances (including anticoagulant therapy), and bleeding diatheses (e.g., hemophilia) and upper gastrointestinal disease (e.g., ulcer, gastritis, hiatus hernia).

While acetaminophen is equal to aspirin in analgesic and antipyretic effectiveness, it is unlikely to produce many of the side effects associated with aspirin and aspirin-containing products. Acetaminophen has thus been recognized as a safe and effective medication in recommended dosage regimens.

G. Ramachander et al. in an article entitled "Effect of Salicylamide and Acetaminophen on Dextromethorphan Hydrobromide Metabolism: Possible Pharmacological Implications" which appeared in The Journal of Pharmaceutical Sciences, 67(6):761-764, June 1978, demonstrated that acetaminophen had an effect on a metabolic fate of dextrorphan, the primary metabolite of dextromethorphan, when studied in vitro in the rat when co-administered with a combination of salicylamide and acetaminophen. Further, Ramachander et al. demonstrate a enhanced antitussive activity of dextromethorphan hydrobromide in a dog when co-administered with a combination of salicylamide in acetaminophen. Ramachander et al. did not study the effects of acetaminophen alone on enhancing the antitussive effect of dextromethorphan.

Dextromethorphan in its hydrobromide salt is a demonstrated safe and effective non-narcotic cough suppressant for the temporary relief of coughing. Dextromethorphan hydrobromide is available in a wide variety of cough and cough/cold medications including, for example, TYLENOL brand cold medication which includes a combination of acetaminophen, dextromethorphan hydrobromide, chlorpheniramine maleate, and pseudoephedrine.

The potential of dextromethorphan abuse has been cited, for example, at FDA's Drug Abuse Advisory Committee Meeting of August 6, 1990 which is reported in FDC Reports of August 13, 1990 at pages 9 and 10. It is reported that dextromethorphan containing cough/cold products may be abused for their apparent psychoactive affects. In light of such potential for abuse it may be beneficial to limit the dosages of dextromethorphan suggested for administration to patients or available in dosage forms.

Potentiation of dextromethorphan as an antitussive would serve not only to increase the products effectiveness for treating a cough but also possibly reduce the dosage amounts that might be available for potential abuse. It has now been found that acetaminophen provides antitussive potentiation to dextromethorphan when co-administered to human patients.

## Summary of the Invention

It is therefore an object of the invention to provide novel methods of use of acetaminophen to potentiate the antitussive effectiveness of dextromethorphan.

Additional objects and advantages of the invention will be set forth, in part, in the description which follows and in part will be obvious from this description, or may be learned by practice of the invention. The objects and advantages of the invention are realized and obtained by means of the methods, and the combinations particularly pointed out in the appended claims.

To achieve the objects in accordance with the purposes of the invention, as embodied and fully described herein, the invention comprises a method of enhancing the antitussive effectiveness of dextromethorphan by administering to a patient dextromethorphan with an antitussive potentiating amount of acetaminophen. In preferred embodiments of the invention the dextromethorphan is administered in a dosage amount of from 0.5 to 1.0 mg. and the acetaminophen is administered in an amount of from 10 to 15 mg. per kg. of body weight of a patient. In other preferred embodiments of the invention the dosage amount of dextromethorphan is in an amount of from 20 to 40 mg. and the acetaminophen is administered in an amount of from 650 to 1000 mg. to an adult patient.

As embodied and fully described herein, the invention further comprises an improved antitussive pharmaceutical composition consisting of an antitussive effective amount of dextromethorphan, an antitussive effective amount of acetaminophen and pharmaceutical excepients. In further embodiments the invention provides a pharmaceutical composition for providing an enhanced antitussive effect to a patient suffering from a cough consisting essentially of an antitussive effective amount of dextromethorphan and an antitussive enhancing effective amount of acetaminophen.

As embodied and fully described herein, the invention further comprises a method of reducing an antitussive effective dosage of dextromethorphan by administering concurrently therewith an antitussive potentiating effective amount of acetaminophen.

It is to be understood that both the foregoing general and the following detailed description are exemplary and explanatory only and are not intended to be restrictive of the invention as claimed.

Detailed Description of the Preferred Embodiments of the Invention

Reference will now be made in detail to present preferred embodiments of the invention, examples of which are illustrated in the following example section.

In accordance with the invention novel methods are provided to achieve the object in accordance with the purposes of the invention, as embodied and fully described herein. The invention provides a method of enhancing the antitussive effectiveness of dextromethorphan by administering to a patient an antitussive effective amount of dextromethorphan in combination with an antitussive potentiating amount of acetaminophen. For the purposes of the present invention an antitussive effective amount of dextromethorphan is defined as a dosage amount of from 0.5 to 1.0 mg. per kg. of body weight of the patient or about 20 to 40 mgs. when administered to an adult patient. For the purposes of the present invention an effective antitussive potentiating amount of acetaminophen is from 10 to 15 mgs. per kg. of body weight of the patient or from 650 to 1000 mgs. for an adult patient.

In accordance with the invention an improved antitussive pharmaceutical composition is provided which consists of an antitussive effective amount of dextromethorphan, an antitussive potentiating amount of acetaminophen and non-active pharmaceutical excipients. These pharmaceutical excipients can include flavorings, colorings, diluents, bulking agents, binders, disintegrants, wicking agents, lubricants and tableting aids. These excipients can also include various gums, suspending agents and other pharmaceutically acceptable excipients as will be known to those skilled in the art.

The pharmaceutical composition of the invention can provide an enhanced antitussive effect to a patient suffering from a cough as compared to the administration of dextromethorphan alone by administering an antitussive effective amount of dextromethorphan in combination with an antitussive enhancing effective amount of acetaminophen. This antitussive enhancing effective amount of acetaminophen is analogous to the potentiating amount of acetaminophen described above.

In accordance with the invention the antitussive effective dosage of dextromethorphan may be reduced by administering dextromethorphan concurrently with an antitussive potentiating effective amount of acetaminophen. This reduction in effective dosage amounts of dextromethorphan provides multiple benefits of reducing the amount of pharmaceutical actives ingested by a patient to relieve cough and also reduces the dosage amount of dextromethorphan available in a dosage form for potential abuse.

The antitussive potentiating effect of acetaminophen in human patients has been demonstrated by the present inventors in human clinical trials which are discussed in the examples section. The invention will now be illustrated by examples. The examples are not intended to be limiting of the scope of the present invention. In conjunction with the detailed and general description above, the examples provide further understanding of the present invention and outline a process for reducing compositions of the invention.

The following examples represent preferred embodiments of the combinations, processes and methods of the invention for satisfying the stated objects of the invention.

Examples

The following examples are prepared using conventional excipients and methods known to those skilled in the art as for example is taught in Remington's Pharmaceutical Sciences, 17th Ed., Mack Pub. Co. 1985, Part 8 "Pharmaceutical Preparations And Their Manufacture" at Pp 1409-1662. The entire disclosure of which is hereby incorporated herein by reference.

Example I

Cough Liquid Dose Formulation
30 mg Dextromethorphan
1000 mg Acetaminophen
Pharmaceutical excipients q.s. to 20 ml per dose

Example II

Cough/Cold Pediatric Liquid Dose Formulation
160 mg Acetaminophen
1 mg Chlorpheniramine
15 mg Pseudoephedrine
5 mg Dextromethorphan
Pharmaceutical excipients q.s. to 5 ml per dose

Example III

Cough/Cold Pediatric Chewable Tablet or Caplet
160 mg Acetaminophen
1 mg Chlorpheniramine
15 mg Pseudoephedrine
5 mg Dextromethorphan
Pharmaceutical excipients including taste masking and tableting components to provide 350 mg tablets

Example IV

Cough with Decongestant Liquid Dose Formulation
30 mg Dextromethorphan
1000 mg Acetaminophen
60 mg Pseudoephedrine
Pharmaceutical excipients q.s. to 20 ml per dose

Example V

Night time cough product dose formulation
1000 mg Acetaminophen
30 mg Dextromethorphan
25 mg Diphenydramine

Example VI

Night time cough product dose fomulation in a liquid or solid dosage form.
Same as Example V but substitute 650 mg for 1000 mg acetaminophen and 50 mg for 25 mg of diphenydramine

Example VII

Cough/Expectorant product dose formulation
1000 mg Acetaminophen
30 mg Dextromethorphan
100 mg Quaifenesin

Example VIII

Same as Example VII but substitute 650 mg for 1000 mg of acetaminophen.

Clinical Demonstration of the Potentiating Effect of Acetaminophen on Dextromethorphan

A comparison of the antitussive activity of 1000 mg. APAP/30 mg. dextromethorphan liquid, 30 mg. dextromethorphan liquid, placebo liquid, and 1000 mg. APAP liquid in volunteers with cough induced by a 5% citric acid aerosol stimulus. The test was conducted in a double-blind study to determine the relative antitussive effects of the following formulations: (1) 1000 mg. of acetaminophen (APAP)/30 mg. dextromethorphan liquid, (2) 30 mg. dextromethorphan liquid, (3) placebo liquid, and (4) 1000 mg. acetaminophen liquid. The experimental design was a double-blind four period crossover using 32 normal and healthy subjects.

This study accumulated data on the efficacy and duration of activity of the following formulations: 1000 mg. APAP/30 mg. dextromethorphan liquid, 30 mg. dextromethorphan liquid, placebo liquid and 1000 mg. APAP liquid in normal, healthy subjects with cough intermittently induced by a 5% citric acid aerosol stimulus. All of the subjects exhibited a significant degree of cough when challenged with a citric acid aerosol stimulus.

The 32 subjects were tested once weekly for 4 consecutive weeks. During each of the 4 drug administration periods, 8 volunteers received 1000 mg. APAP/30 mg. dextromethorphan liquid, 8 received 30 mg. dextromethorphan liquid, 8 received placebo liquid and 8 received 1000 mg. APAP liquid. The order in which medications were dispensed was determined prior to the study according to a randomization schedule using 4 different sequences of treatment administration. By the end of the study, each subject was tested with single doses of each of the 4 test formulations.

On each weekly visit an identical procedure was followed. Each tussigenic challenge was comprised of 5 individual administrations of a 5% citric acid aerosol stimulus. The cough count for each challenge period was calculated as the sum of the cough counts of the five individual administrations. After ensuring that the patient met all entrance requirements, a baseline cough count was performed and if this baseline cough count was between 10 and 15 coughs, the subject took his assigned treatment. The citric acid challenge procedure was then repeated at 60, 120, 240, 360 and 480 minutes from that time.

Study medication consisted of the following four treatments:
1) 1000 mg. APAP/30 mg. dextromethorphan liquid in 30 ml.
2) 30 mg. dextromethorphan liquid in 30 ml.
3) Placebo liquid - each container of placebo medication allowed for the single dose administration of 30 ml. of placebo medication.
4) 1000 mg. APAP liquid in 30 ml.

The pure dextromethorphan formulation contained alcohol 6% volume-by-volume (v/v) and polyethylene glycol 6 grams percent while the three remaining formulations contained alcohol 7.5% v/v and polyethylene glycol 15 grams percent.

A two-way analysis of variance was performed on the baseline cough count to test for any significant differences among the four testing weeks. The results showed no significant differences in baseline cough count among the four weeks (P = .1656). These results are summarized in Table I.

## Table I

### Mean Baseline Cough Counts

| Week 1 | Week 2 | Week 3 | Week 4 |
|--------|--------|--------|--------|
| 11.19 | 10.84 | 11.09 | 11.12 |

Another two-way analysis of variance was performed on the baseline cough counts to test for any significant differences among the four drugs. The results showed a nearly significant difference (P = .0536). For this reason an adjustment was made for initial cough count in subsequent analyses. The means for each drug are shown in Table II.

## Table II


### Mean Baseline Cough Counts


    (1) **APAP/Dextromethorphan**:   11.19

    (2) **Dextromethorphan**    :   10.94

    (3) **Placebo**           :   11.25

    (4) **APAP**             :   10.875


Treatment effects were measured by considering the difference between baseline cough count and challenge time cough count at hours 1, 2, 4, 6, and 8.

The results of these analyses indicated a highly significant difference among the treatments. Duncan's multiple range procedure with an overal 95% significance level indicated that APAP/dextromethorphan was significantly superior to all other treatments.

Duncan's multiple range procedure with an overall 95% significance level indicated that APAP/dextromethorphan was significantly superior to all other treatments, dextromethorphan was significantly superior to placebo and APAP, and placebo and APAP were not significantly different from each other for all of the variables considered. Table III displays the mean cough counts and the mean differences from baseline at each of the challenge times for each treatment.

TABLE III

Mean Cough Counts and Differences From Baseline

| Time | APAP/Dextromethorphan | | Dextromethorphan | | Placebo | | APAP | |
|---|---|---|---|---|---|---|---|---|
| | Mean Cough Count | Mean Differences | Mean Cough Count | Mean Differences | Mean Cough Count | Mean Differences | Mean Cough Count | Mean Differences |
| 0 | 11.19 | | 10.94 | | 11.25 | | 10.88 | |
| 1 | 8.56 | 2.63 | 9.03 | 1.91 | 11.09 | .16 | 10.56 | .31 |
| 2 | 7.00 | 4.19 | 8.22 | 2.72 | 11.19 | .06 | 10.59 | .28 |
| 4 | 7.00 | 4.19 | 7.91 | 3.03 | 10.88 | .38 | 10.50 | .38 |
| 6 | 8.06 | 3.13 | 8.81 | 2.13 | 11.00 | .25 | 10.63 | .25 |
| 8 | 8.75 | 2.44 | 9.44 | 1.50 | 10.97 | .28 | 10.91 | -.03 |

Clinical Comparison II

A clinical was carried out in a similar manner to the clinical demonstration described above. The preparations tested in healthy adult subjects as well as summary statistics and results obtained thereby are indicated below.

7

Preparations Compared By Citric Acid
<u>Aerosol Induced Cough Procedure</u>

|  | Abbreviation for Tables |
|---|---|
| <u>Acetaminophen/dextromethorpan - 30 ml.</u> | ACET1000/DEX30 |
| Analgesic - Acetaminophen 1000 mg. | |
| Cough Suppressant - Dextromethorpan Hydrobromide 30 mg. | |
| <u>Acetaminophen/Dextromethorphan - 15 ml.</u> | ACET650/DEX30 |
| Analgesic - Acetaminophen 650 mg. | |
| Cough Suppressant - Dextromethorphan Hydrobromide 30 mg. | |

Acetaminophen/Dextromethorphan/

Pseudoephedrine - 15 ml.          ACET650/DEX30/

PSEUD60

Analgesic - Acetaminophen 650 mg.

Cough Suppressant - Dextromethorpahn

Hydrobrombide 30 mg.

Nasal Decongestant - Pseudoephedrine

Hydrochloride 60 mg.


Dextromethorphan - 15 ml.          DEX30

Cough Suppressant - Dextromethorphan

Hydrobromide 30 mg.


Placebo          PLACEBO


Tussi-Organidin - 10 ml.          TUSSI-ORG

Iodinated Glycerol 30 mg./5 ml.

Cough Suppressant - Codeine Phosphate

10 mg./5 ml.


Robitussin DM® - 10 ml.          ROB-DM®

Expectorant - Guaifenesin 200 mg.

Cough Suppressant - Dextromethorphan/

Hydrobromide 20 mg.


Formula 44D® - 15 ml.          FORM-44D®

Expectorant - Guaifenesin 200 mg.

Cough Suppressant - Dextromethorphan/

Hydrobromide 30 mg.

Nasal Decongestant - Pseudoephedrine

Hydrochloride 60 mg.


The results of this antitussive screening is provided below in Table IV.

TABLE IV

ANTITUSSIVE SCREENING: CITRIC ACID AEROSOL TECHNIQUE

Summary Statistics: Coughs per 5 Inhalations

| Treatment | Hour 0 | | Hour 1 | | Hour 2 | | Hour 4 | | Hour 6 | | Hour 8 | |
|---|---|---|---|---|---|---|---|---|---|---|---|---|
| | Mean | Std. Err. | Mean | Std. Err. | Mean | Std. Err. | Mean | Std. Err. | Mean | Std. Err. | Mean | Std. Err. |
| ACET 1000/DEX 30 | 11.5 | 0.21 | 8.6 | 0.21 | 7.9 | 0.20 | 8.1 | 0.22 | 9.2 | 0.22 | 9.7 | 0.23 |
| ACET 650/DEX 30 | 11.6 | 0.21 | 9.2 | 0.21 | 8.1 | 0.23 | 8.6 | 0.21 | 9.5 | 0.21 | 10.3 | 0.21 |
| ACET 650/DEX 30/ PSEUD 60 | 11.6 | 0.21 | 9.2 | 0.22 | 7.9 | 0.21 | 8.5 | 0.21 | 9.4 | 0.21 | 10.2 | 0.21 |
| DEX 30 | 11.6 | 0.21 | 9.0 | 0.22 | 8.6 | 0.21 | 8.7 | 0.22 | 9.6 | 0.21 | 10.5 | 0.20 |
| FORM 44D® | 11.3 | 0.20 | 8.8 | 0.20 | 8.0 | 0.21 | 8.3 | 0.20 | 8.9 | 0.20 | 10.0 | 0.20 |
| ROB DM® | 11.5 | 0.19 | 9.0 | 0.19 | 8.2 | 0.21 | 8.6 | 0.20 | 9.2 | 0.21 | 10.2 | 0.18 |
| TUSSI-ORG | 11.5 | 0.21 | 9.6 | 0.22 | 9.5 | 0.21 | 9.8 | 0.22 | 10.5 | 0.21 | 10.5 | 0.21 |
| PLACEBO | 11.5 | 0.20 | 11.0 | 0.21 | 10.9 | 0.20 | 10.9 | 0.21 | 10.9 | 0.22 | 10.5 | 0.23 |

Results

The following section summarizes results of this clinical comparison whereby the symbol ">" indicates that the ACET 1000/DEX 30 group had a significantly greater percent reduction in cough count from Hour 0 than the treatment group(s) on the right. The symbol "=" indicates no significant difference:

Hour 1 (Difference ≧ 2.25% is statistically significant)
ACET 1000/DEX 30 > All other treatments

Hour 2 (Difference $\geq$ 2.20% is statistically significant)

ACET 1000/DEX 30 > ROB_DM®, DEX 30, TUSSI-ORG, PLACEBO = ACET 650/DEX 30, ACET 650/DEX 30/ PSEUD 60, FORM-44D®, ROB-DM®

Hour 4 (Difference $\geq$ 2.04% is statistically significant)

ACET 1000/DEX 30 > All other treatments

Hour 6 (Difference $\geq$ 1.87% is statistically significant)

ACET 1000/DEX 30 > DEX 30, TUSSI-ORG, PLACEBO = ACET 650/DEX30/PSEUD 60, FORM-44D®, ROB-DM®

Hour 8 (Difference $\geq$ 1.85% is statistically significant)

ACET 1000/DEX 30 > All other treatments

The results of these clinical studies do indicate that acetaminophen does potentiate the antitussive effect of dextromethorphan in human patients.

The scope of the present invention is not limited by the description, examples and suggested uses herein and modifications can be made without departing from the spirit of the invention. Applications of the methods of the present invention can be accomplished by any suitable therapeutic method and technique as is presently or prospectively known to those skilled in the art. Thus, it is intended that the present invention covers the modifications and variations of this invention provided that they come within the scope of the appended claims and their equivalents.

## Claims

1. An antitussive pharmaceutical composition consisting of an antitussive effective amount of dextromethorphan and an antitussive potentiating amount of acetaminophen.

2. The composition of claim 1, wherein the dextromethorphan and acetaminophen are the only active ingredients in the composition.

3. The composition of claim 1 or claim 2, containing 0.5 to 1.0 mg/kg body weight of dextromethorphan and 10 to 15 mg/kg body weight of acetaminophen.

4. The composition of claim 1 or claim 2, containing 20 to 40 mg of dextromethorphan and 650 to 1000 mg acetaminophen.

5. The composition of claim 4, containing less 30 mg of dextromethorphan.

6. The composition of any one of claims 1 to 5, for use in enhancing the antitussive effectiveness of dextromethorphan.

7. The composition of any one of claims 1 to 5, for use in providing an enhanced antitussive effect to a patient suffering from a cough.

8. The composition of any one of claims 1 to 5, for use in reducing the antitussive effective dose of dextromethorphan.

**European Patent Office**

## EUROPEAN SEARCH REPORT

Application Number

EP 92 30 7474

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | J. PHARM. SCI., vol. 67, no. 6, June 1978, pages 761-764; G. RAMACHANDER et al.: "Effect of salicylamide and acetaminophen on dextromethorphan hydrobromide metabolism: possible pharmacological implications" * Whole article * | 1-8 | A 61 K 31/485 // (A 61 K 31/485 A 61 K 31:165) |
| X | EP-A-0 081 823 (NELSON R & D CO.) * Claims 7,8 * | 1-8 | |
| X | US-A-3 446 891 (A. CAVALLI) * Column 1, lines 27-31; column 2, lines 38-47,54-57; example 2; claims 1,2 * | 1-8 | |
| X | "Rote Liste", 1989, Bundesverband der Pharmazeutischen Industie e.V., Editio Cantor, Aulendorf/Württ, DE * 44016; 44017; 44018 * | 1-8 | |
| X | "Rote Liste", 1991, Bundesverband der Pharmazeutischen Industrie e.V., Editio Cantor, Aulendorf/Würt, DE * 44014; 44017; 44018 * | 1-8 | TECHNICAL FIELDS SEARCHED (Int. Cl.5) A 61 K |

The present search report has been drawn up for all claims

| Place of search | Date of completion of the search | Examiner |
|---|---|---|
| THE HAGUE | 25-11-1992 | ORVIZ DIAZ P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)